# EUROPEAN PATENT APPLICATION

(11) **EP 2 181 682 A2**
(43) Date of publication of application: **05.05.2010**
(21) Application number: 09170865.1
(22) Date of filing: 21.09.2009
(51) Int. Cl.: A61F 15/00

(54) **Pocket-sized adhesive plaster holder device with internal mirror**

(30) Priority: 02.10.2008 IT MI20081753
(71) Applicant: GI.BI.EFFE S.r.l., 20135 Milano (IT)
(72) Inventor: Lo Duca, Rubina, 20141, MILANO (IT)
(74) Representative: Frignoli, Luigi

(57) **Abstract**

A pocket-sized adhesive plaster holder device formed from a punched and crease-lined cardboard blank foldable onto itself to form a casing which is maintained in the closed position by coupling elements provided in a peripheral portion of the cardboard blank, which is able to house and retain one or more plasters applicable to the human skin. The internal surface of the cardboard blank is mirror-finished to enable easy viewing of that part of the face on which the plaster is to be applied.

## Description

The present invention relates to a pocket-sized adhesive plaster holder device having a mirrored internal surface.

Adhesive plasters, usually of small dimensions, are known for application to the facial skin, for example to the lips, nose, cheeks, etc. of the person using the adhesive plaster: some adhesive plasters carry a medical or pharmaceutical substance, but others not.

The usual problem is that of correctly positioning the adhesive plaster in the desired point on the face by the person who, to achieve this, uses a mirror which can also be inserted into the case or box containing the plasters.

Plaster-containing boxes are of relatively large dimensions to house a relatively large number of plasters. Plasters exist which have to be replaced on the human face by new plasters after a predetermined time period, for example every two, six or twelve hours or at other intervals. The person using such plasters has therefore always to have available a box containing the plasters, which may be uncomfortable if the person is travelling or is far from the normal place of stay (for example working in an office) for a time longer than the interval between the plaster application and its replacement with a new plaster.

Adhesive plasters of the type to be used in a relatively large number are contained in boxes from which they can be withdrawn by raising the box lid or in other equivalent ways. If the plaster is to be applied on a well defined region of the user's face, the user must use a mirror to clearly identify this facial region: to facilitate this operation a mirror can be inserted into the box containing the plasters, this generally increasing costs.

US-A-3899077 describes an enclosure for containing adhesive plasters in respective separate seats from which the plasters can be withdrawn for application to the human skin: this operation is very difficult and requires the use of an external mirror if the plaster is to be applied to the user's face or neck.

US 2005/067421 A1 and US 2005/133382 A1 describe boxes for containing articles of various types (such as contact lenses), these boxes being made of rigid material and having a mirror provided on their inner surface: this makes it extremely difficult, inter alia, to use the mirror to clearly see the reflected surface of the face, neck, eyes or the like, on which the article is to be positioned.

An object of the present invention is to provide an adhesive plaster holder device which is pocket-sized, the word "pocket-sized" meaning that the device must be flexible and of small dimensions, to be able to be easily inserted and retained in a pocket of the person's clothes or in a handbag or the like, and preferably is able to contain only a small number of plasters, such that they can be easily withdrawn by the person using them. The main object of the present invention is to provide an adhesive plaster holder device which is pocket-sized (in the aforespecified sense), is of low cost and simple structure, and has at least one portion of its surface which is mirrored.

These and other objects are attained by an adhesive plaster holder device, characterised by being formed from a one-piece punched and crease-lined cardboard blank, foldable onto itself about a folding line thereof to form a pocket, the cardboard blank being shaped to define elements manually movable into a position in which they mutually engage to securely retain the cardboard blank folded onto itself, at least one portion of the cardboard surface being mirrored.

Preferably said mirrored surface of the device is provided on that part of the cardboard blank which is intended to remain facing the interior of the device when this is in its closed position.

Again preferably, cuts or small elongated apertures are provided in the cardboard blank along said folding line.

A preferred but non-limiting embodiment of the adhesive plaster holder device is described hereinafter with reference to the accompanying drawing, in which:
Figure 1 is a plan view of a punched and crease-lined cardboard blank usable for shaping a adhesive plaster holder device;
Figure 2 is similar to Figure 1 but also shows a mirror-reflecting sheet applied to the flat surface of the cardboard blank;
Figure 3 shows the cardboard blank of Figure 2 folded at 90º onto itself about its folding line;
Figure 4 is similar to Figure 3 but differs in that it shows two adhesive plasters inserted into the cardboard blank; and
Figure 5 shows the cardboard blank with its two parts completely folded onto themselves and coupled together to securely retain the adhesive plasters inserted therein.

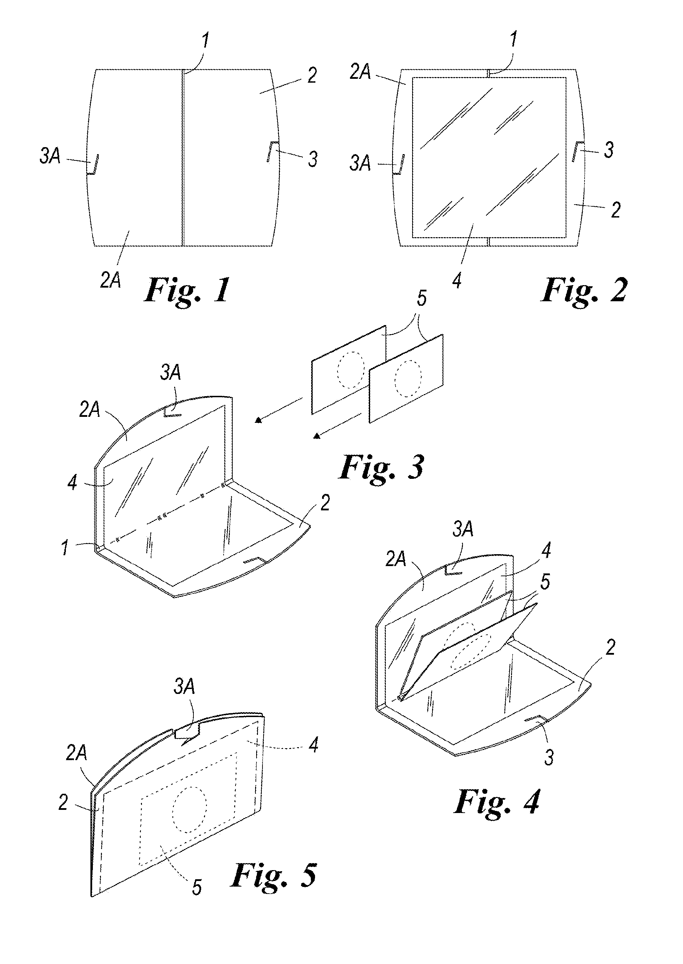

Figure 1 shows a plan view of a cardboard blank in which a creasing line 1 divides the cardboard blank into two specular parts 2 and 2A respectively, in the outer edges of which two small cuts are provided to define flexible appendices 3 and 3A respectively.

It will be immediately noted that the creasing line 1 can be formed from two (or more than two) parallel spaced-apart folding lines, and that separate aligned cuts or small elongated apertures (not shown for simplicity in the drawing) can be provided along the creasing line to facilitate the folding (described hereinafter) of the cardboard blank and possibly to maintain the two parts 2 and 2A of the cardboard blank spaced apart within the closed device shown in Figure 5 (in order to facilitate the containing of the adhesive plasters.

Fixed (for example glued) onto the smooth surface of the cardboard blank (Figure 2) there is a thin polyester sheet 4 of thickness 100 micron having its free surface (that facing upwards in Figure 2) of mirror-reflecting type: evidently the polyester sheet could instead be applied to only one of the two parts 2 or 2A of the cardboard blank, and its mirrored surface could be obtained in a different manner, for example by silver-plating or by surface varnishing.

When the cardboard blank is folded onto itself (as shown in Figure 3), small adhesive plasters 5 of known type (two are shown in the drawing) can be inserted between the two parts 2 and 2A of the cardboard blank, to assume the attitude observable from Figure 4.

The two parts 2 and 2A of the cardboard blank can then be completely folded onto each other (to hence clamp the plasters 5 between them) and be retained in this position by flexure and by the insertion of each of the flexible appendices 3, 3A into the cut which bounds the other flexible appendix, as can be seen from Figure 5.

In this manner an adhesive plaster holder device is obtained which is of small dimensions (and can hence be defined as "pocket-sized"), and contains a small number of plasters which can be easily extracted from the device and which can finally be easily and correctly applied to any point of the user's face by making use of the mirrored surface 4.

## Claims

1. A pocket-sized adhesive plaster holder device, **characterised by** being formed from a one-piece punched and crease-lined cardboard blank, (2, 2A) foldable onto itself about a folding line (1) thereof to form a pocket, the cardboard blank being shaped to define elements (3, 3A) manually movable into a position in which they mutually engage to securely retain the cardboard blank folded onto itself, at least one portion (4) of the cardboard surface being mirrored.

2. An adhesive plaster holder device as claimed in claim 1, **characterised in that** said mirrored surface (4) of the device is provided on that part of the cardboard blank (2, 2A) which is intended to remain facing the interior of the device when this is in its closed position.

3. An adhesive plaster holder device as claimed in claim 1 or 2, **characterised in that** cuts or small elongated apertures are provided in the cardboard blank along said folding line (1).
